# EUROPEAN PATENT SPECIFICATION

(11) **EP 4 404 987 B1**
(45) Date of publication and mention of the grant of the patent: **29.10.2025**
(21) Application number: 22790573.4
(22) Date of filing: 21.09.2022
(51) Int. Cl.: A61L 27/20, A61K 9/00, A61K 9/06, A61K 31/18, A61K 31/4164, A61K 31/496, A61K 31/7048, A61K 47/36, A61L 27/22, A61L 27/38, A61L 27/52, A61L 27/54, A61L 27/58

(54) **ODONTOSTOMATOLOGICAL PREPARATION COMPRISING HYALURONIC ACID, ANTIBIOTICS AND GROWTH FACTORS**
ODONTOSTOMATOLOGISCHE ZUBEREITUNG MIT HYALURONSÄURE, ANTIBIOTIKA UND WACHSTUMSFAKTOREN
PRÉPARATION ODONTOSTOMATOLOGIQUE COMPRENANT DE L'ACIDE HYALURONIQUE, DES ANTIBIOTIQUES ET DES FACTEURS DE CROISSANCE

(30) Priority: 21.09.2021 IT 202100024257
(43) Date of publication of application: 31.07.2024
(73) Proprietor: Università degli Studi di Torino, 10124 Torino (IT)
(72) Inventor: ALOVISI, Mario, 10123 TORINO (TO) (IT); MANDRAS, Narcisa, 10123 TORINO (TO) (IT); ROANA, Janira, 10123 TORINO (TO) (IT); ARPICCO, Silvia, 10123 TORINO (TO) (IT); CUFFINI, Annamaria, 10123 TORINO (TO) (IT); BERUTTI, Elio, 10123 TORINO (TO) (IT)
(74) Representative: Studio Torta S.p.A.
(86) International application number: PCT/IB2022/058917
(87) International publication number: WO 2023/047297

(56) References cited:
- MANDRAS NARCISA ET AL: "Evaluation of the Bactericidal Activity of a Hyaluronic Acid-Vehicled Clarithromycin Antibiotic Mixture by Confocal Laser Scanning Microscopy", APPLIED SCIENCES, vol. 10, no. 8, 13 April 2020 (2020-04-13), pages 761, XP055915330, DOI: 10.3390/app10080761
- DATABASE WPI Week 201562, Derwent World Patents Index; AN 2015-47344X, XP002806306
- PETTA D ET AL: "Hyaluronic acid as a bioink for extrusion-based 3D printing", vol. 12, no. 3, 28 May 2020 (2020-05-28), pages 032001, XP055915864, Retrieved from the Internet <URL:https://iopscience.iop.org/article/10.1088/1758-5090/ab8752> DOI: 10.1088/1758-5090/ab8752

## Description

### Cross-Reference to Related Applications

This Patent Application claims priority from Italian Patent Application No. 102021000024257 filed on September 21, 2021.

### Technical Field

The present invention relates to a biodegradable odontostomatological preparation that is used in the disinfection, healing and regeneration of oral tissues, for example for endodontic, periodontal, surgical and implant applications in the dental field.

### Background of the Invention

Caries and periodontitis are among the most widespread infectious diseases in the world. A recent epidemiological analysis has shown that more than 20% of the world population suffers from chronic periodontitis due to a recurrent bacterial infection, while 37% of children and 58% of adults experience caries in deciduous or permanent teeth.

Apical periodontitis due to an intracanal infection following pulpal necrosis affects about 30% of the adult population and more than 25 million endodontic treatments are performed annually in the United States alone.

Since these pathologies have an infectious aetiology, thorough disinfection of the periodontal defect or of the root canal system is essential for healing.

Modern regenerative dentistry aims to promote the formation of new vital tissue similar to that destroyed by the pathology. This approach, which has recently been proposed for endodontics as well as for periodontology and oral and implant surgery, is based on three fundamental aspects: stem cells, signal molecules and the scaffold, i.e. the resorbable scaffold necessary for healing and maintaining space for regenerated tissue. The scaffold has the task of providing a substrate dedicated to stem cells for their multiplication and differentiation, as well as promoting their regenerative activity through the supply of numerous growth factors, including FGF, TGFbeta and VEGF. However, this regenerative process can only take place after adequate disinfection and thorough elimination of all pathogenic bacteria that caused the infection. Several disinfectant solutions have been proposed in endodontics, periodontics and oral surgery.

To date, therefore, the endodontic, periodontal and surgical treatment envisages a first step of disinfection of the site to be treated and the subsequent implantation of a scaffold that enables stem cell differentiation and tissue repair. CN 104 548 212 A discloses a composition for promoting pulp and dentin regeneration composition comprising PDGF and a hyaluronic acid scaffold.

The need for products that combine the step of broad-spectrum disinfection and active regeneration of the tissues involved in an endodontic, surgical, implant or periodontal infection is therefore felt in the art.

### Summary

Therefore, aim of the present invention is to provide a system that allows both the disinfection and the repair of the oral tissues for endodontics as well as for periodontics and the oral and implant surgery to be combined in a single step.

This aim is achieved by means of a bioresorbable odontostomatological preparation according to claim 1 and the odonstostomatological preparation for use in the disinfection, healing and regeneration of oral tissues according to claim 8.

### Brief Description of the Drawings

The present invention will now be described in detail with reference to the figures of the accompanying drawings, wherein:
- Figure 1 shows the evaluation of the bacterial load in three samples of the invention as a function of the incubation time, at each time interval (1, 2, 4, 24 and 48 hours).
- Figure 2 shows the evaluation of the bacterial load in the three samples as a function of the incubation time from the point of view of the individual sample.

### Description of Embodiments

According to a first aspect of the present invention there is provided a biodegradable odontostomatological preparation comprising a three-dimensional gel of hyaluronic acid having a molecular weight between 50 kDa and 2000 kDa incorporating an antibiotic composition comprising clarithromycin, ciprofloxacin and metronidazole and tissue growth factors selected from the group consisting of PDGF (Platelet-Derived Growth Factor), VEGF (Vascular Endothelial Growth Factor), FGF (Fibroblast Growth Factor) and TGF (Transforming Growth Factor).

Preferably, the molecular weight of the hyaluronic acid is between 50 and 2000 kDa, more preferably 1500 and 1800 kDa.

"Odontostomatological preparation" is defined as a biocompatible preparation that can be used in contact with the hard and soft tissues present within the oral cavity and all contiguous and adjacent anatomical structures.

According to one embodiment, the three-dimensional gel of hyaluronic acid comprises from 0.1 to 5% by weight of hyaluronic acid on the total weight of the gel, preferably from 0.5 to 2% by weight, the remaining part being water.

Preferably, metronidazole is present in a concentration between 1.0 and 2.5 % by weight on the total weight of the composition.

Ciprofloxacin, on the other hand, is present in a concentration between 0.1 and 2.5 % by weight on the total weight of the composition.

Preferably, clarithromycin is present in a concentration between 1.0 and 3.5 % by weight on the total weight of the composition.

The disinfectant odontostomatological scaffold according to the invention comprises tissue growth factors.

"Tissue growth factors" are defined as all proteins capable of stimulating cell proliferation and/or differentiation.

The odontostomatological preparation according to the invention has proved particularly advantageous when used in the disinfection, healing and regeneration of oral tissues.

"Oral tissues" are defined as all hard and soft tissues present within the oral cavity and the contiguous and adjacent anatomical structures.

Advantageously, the present invention is designed so as to actively participate in the step of healing and regeneration as well as of endodontic, periodontal and surgical disinfection in the dental and implant field. Some antibiotic and disinfectant mixtures have been described in the literature, which do not have the characteristics of resorbable scaffolds, but only proven antibacterial efficacy. The combination of the antibiotics metronidazole, ciprofloxacin and clarithromycin has proven to be very effective against endodontic and periodontal bacteria. Indeed, clarithromycin has a good tissue distribution (especially in bone and periodontal tissue, in the lung parenchyma and in the first tracts of the digestive and respiratory system) and is a valuable adjunct to non-surgical periodontal therapy for the treatment of chronic periodontitis. However, there are no odontostomatological products capable of combining regenerative and antibacterial activities for oral tissues.

Hyaluronic acid has the characteristics to be used both as a biocompatible vehicle thanks to its ability of incorporating growth factors and of being able to give rise to systems with different viscosities both for conveying antibacterial molecules modulating their release. In fact, the preparation of the invention can be controlled in its final form and density on the basis of the field of application: in this way it will be possible to adapt the preparation in a personalized manner according to the particular clinical situation. In one embodiment, the final viscosity of the preparation of the invention is therefore variable.

It may further comprise at least one radiopaque agent selected from the group consisting of acrylic polymers, apatite, inorganic salts and metal derivatives.

The preparation of the invention proposes the synergistic combination of the antibacterial capacities of disinfectant molecules effective against broad-spectrum oral bacteria together with the characteristics of prolonged release, space maintenance and cellular trophism of hyaluronic acid at different molecular weights.

The preparation of the invention allows not only the thorough disinfection of the site, but also the recall and multiplication of the host stem cells in one go through the release of growth factors and bioactive molecules capable of stimulating tissue regeneration (e.g. TGFbeta, FGF, VEGF and the like). In practice, a single low-cost product allows two fundamental steps for regeneration in odontostomatology and can also be personalized in the form and in the final volume.

The preparation of the invention can be used in dental applications comprising tissue healing following surgical procedures and pathologies with bacterial infectious aetiology. The most considered disciplines are all those present within medical and surgical odontostomatology, such as endodontics, oral and maxillofacial surgery, periodontology and implantology.

Further characteristics of the present invention will become apparent from the following description of some merely illustrative and non-limiting examples.

### Examples

### EXAMPLE 1.

### A. Materials and Methods

The realization of the preparation of the invention is based on the use of sodium hyaluronate in powder with a molecular weight between 1500 and 1800 KDa. The compound in the 1% concentration has a viscosity equal to 2500-5000 cPs. The following is a description of the procedure to obtain the preparation of the invention: 10 ml of MilliQ water were added to 50, 100 or 200 mg of sodium hyaluronate and mixed gently until the final preparation was obtained. Active products (antibiotic molecules and growth factors) were added to this preparation.

The evaluation of the antimicrobial activity of the preparation of the invention was carried out using the time-kill technique. A bacterial culture of *Enterococcus faecalis* ATCC 29212 at a concentration equal to 10⁷ CFU/ml was contacted with three samples of the preparation of the invention containing different percentages of hyaluronic acid (HA) (0.5%, 1% and 2%) and incubated at predetermined times (1, 2, 4, 24 and 48 hours) at 37°C under a 5% CO₂ enriched atmosphere. More precisely, the different samples of the preparation of the invention contained:
- from 50 to 90 µl (2560/1) of metronidazole + 20-50 µl (1280/1) of ciprofloxacin + 80-120 µl (1280/1) of clarithromycin + 3-6 ml of three-dimensional gel of hyaluronic acid (0.5%) (in Table 1 sample denominated 0.5%);
- from 50 to 90 µl (2560/1) of metronidazole + 20-50 µl (1280/1) of ciprofloxacin + 80-120 µl (1280/1) of clarithromycin + 3-6 ml of three-dimensional gel of hyaluronic acid (1%) (in Table 1 sample denominated 1%);
- from 50 to 90 µl (2560/1) of metronidazole + 20-50 µl (1280/1) of ciprofloxacin + 80-120 µl (1280/1) of clarithromycin + 3-6 ml of three-dimensional gel of hyaluronic acid (2%) (in Table 1 sample denominated 2%).

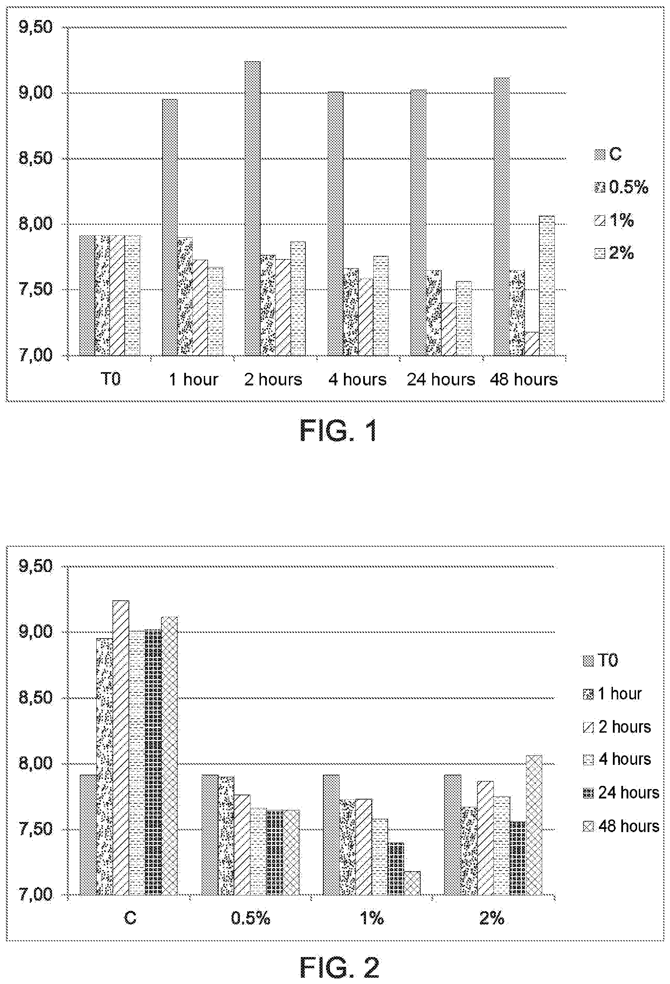

All the preparations contained a predetermined amount of cellular growth factors, in particular FGF, TGF beta, VEGF and PDGF and similar at 2x10⁷ unit/mg. In parallel, a control sample, denominated C, containing only the bacterial culture was set up. After incubation for 24 hours at 37°C under a 5% CO₂ enriched atmosphere, the colonies were counted and the microbial load was expressed as CFU/ml. The results are shown in Table 1 and Figures 1 and 2.

**Table 1**

| Bacterial load | | | | |
|---|---|---|---|---|
| time | C | 0.5 % | 1 % | 2 % |
| 0 hour | 7.92 | 7.92 | 7.92 | 7.92 |
| 1 hour | 8.95 | 7.90 | 7.73 | 7.67 |
| 2 hours | 9.24 | 7.76 | 7.73 | 7.87 |
| 4 hours | 9.01 | 7.66 | 7.58 | 7.75 |
| 24 hours | 9.02 | 7. 64 | 7.40 | 7.56 |
| 48 hours | 9.11 | 7.65 | 7.18 | 8.06 |

After one, two, four, twenty-four and forty-eight hours of contact, in the presence of the preparation of the invention the initial bacterial load showed a significant decrease. The evaluation of the antimicrobial efficacy highlights the presence of bacteriostatic activity.

In addition, the antibiotic-sensitivity study on the solid culture medium, performed in order to evaluate the sensitivity profile of the three antibiotic molecules within the preparation of the invention, showed the presence of an extensive inhibition halo due to drug-induced inhibition of the bacterial growth in the presence of the preparation. The result derives from the formation of a concentration gradient that decreases in a centrifugal direction and whose measured diameter is closely related to the sensitivity of the bacterial strain towards the tested molecules.

## Claims

1. Biodegradable odontostomatological preparation comprising a three-dimensional gel of hyaluronic acid having a molecular weight between 50 kDa and 2000 kDa incorporating
- an antibiotic composition comprising clarithromycin, ciprofloxacin and metronidazole, and
- tissue growth factors selected from the group consisting of PDGF (Platelet-Derived Growth Factor), VEGF (Vascular Endothelial Growth Factor), FGF (Fibroblast Growth Factor) and TGF (Transforming Growth Factor).

2. Odontostomatological preparation according to claim 1, **characterized in that** said three-dimensional gel of hyaluronic acid comprises from 0.1 to 5% by weight of hyaluronic acid on the total weight of the gel.

3. Odontostomatological preparation according to claim 1, **characterized in that** it comprises from 1.0 to 2.5% by weight of metronidazole on the total weight of the composition.

4. Odontostomatological preparation according to claim 1, **characterized in that** it comprises from 0.1 to 2.5% by weight of ciprofloxacin on the total weight of the composition.

5. Odontostomatological preparation according to claim 1, **characterized in that** it comprises from 1.0 to 3.5% by weight of clarithromycin on the total weight of the composition.

6. Odontostomatological preparation according to claim 1, **characterized in that** it comprises at least one radiopaque agent selected from the group consisting of acrylic polymers, apatite, inorganic salts and metal derivatives.

7. Method for manufacturing the odontostomatological preparation according to any one of the preceding claims, comprising the step of 3D printing said preparation.

8. Odontostomatological preparation according to any one of the preceding claims for use in the disinfection, healing and regeneration of oral tissues.

## Patentansprüche

1. Biologisch abbaubares odontostomatologisches Präparat, das ein dreidimensionales Gel aus Hyaluronsäure mit einem Molekulargewicht zwischen 50 kDa und 2000 kDa umfasst, das Folgendes enthält
- eine antibiotische Zusammensetzung, die Clarithromycin, Ciprofloxacin und Metronidazol umfasst, und
- Gewebewachstumsfaktoren, ausgewählt aus der Gruppe bestehend aus PDGF (Platelet-Derived Growth Factor), VEGF (Vascular Endothelial Growth Factor), FGF (Fibroblast Growth Factor) und TGF (Transforming Growth Factor).

2. Odontostomatologisches Präparat nach Anspruch 1, **dadurch gekennzeichnet, dass** das dreidimensionale Gel aus Hyaluronsäure 0,1 bis 5 Gew.-% Hyaluronsäure, bezogen auf das Gesamtgewicht des Gels, umfasst.

3. Odontostomatologisches Präparat nach Anspruch 1, **dadurch gekennzeichnet, dass** es 1,0 bis 2,5 Gew.-% Metronidazol, bezogen auf das Gesamtgewicht der Zusammensetzung, umfasst.

4. Odontostomatologisches Präparat nach Anspruch 1, **dadurch gekennzeichnet, dass** es 0,1 bis 2,5 Gew.-% Ciprofloxacin, bezogen auf das Gesamtgewicht der Zusammensetzung, umfasst.

5. Odontostomatologisches Präparat nach Anspruch 1, **dadurch gekennzeichnet, dass** es 1,0 bis 3,5 Gew.-% Clarithromycin, bezogen auf das Gesamtgewicht der Zusammensetzung, umfasst.

6. Odontostomatologisches Präparat nach Anspruch 1, **dadurch gekennzeichnet, dass** es mindestens ein röntgenopakes Mittel umfasst, das aus der Gruppe ausgewählt ist, die aus Acrylpolymeren, Apatit, anorganischen Salzen und Metallderivaten besteht.

7. Verfahren zur Herstellung des odontostomatologischen Präparats nach einem der vorhergehenden Ansprüche, das den Schritt zum 3D-Drucken des Präparats umfasst.

8. Odontostomatologisches Präparat nach einem der vorhergehenden Ansprüche zur Verwendung bei der Desinfektion, Heilung und Regeneration von Mundgewebe.

## Revendications

1. Préparation odontostomatologique biodégradable comprenant un gel tridimensionnel d'acide hyaluronique ayant un poids moléculaire compris entre 50 et 2000 kDa incorporant
- une composition antibiotique comprenant de la clarithromycine, de la ciprofloxacine et du métronidazole, et
- des facteurs de croissance tissulaire choisis dans le groupe constitué par le PDGF (facteur de croissance dérivé des plaquettes), le VEGF (facteur de croissance endothélial vasculaire), le FGF (facteur de croissance de fibroblastes) et le TGF (facteur de croissance transformant).

2. Préparation odontostomatologique selon la revendication 1, **caractérisée en ce que** ledit gel tridimensionnel d'acide hyaluronique comprend de 0,1 à 5 % en poids d'acide hyaluronique par rapport au poids total du gel.

3. Préparation odontostomatologique selon la revendication 1, **caractérisée en ce qu'**elle comprend de 1 à 2,5 % en poids de métronidazole par rapport au poids total de la composition.

4. Préparation odontostomatologique selon la revendication 1, **caractérisée en ce qu'**elle comprend de 0,1 à 2,5 % en poids de ciprofloxacine par rapport au poids total de la composition.

5. Préparation odontostomatologique selon la revendication 1, **caractérisée en ce qu'**elle comprend de 1 à 3,5 % en poids de clarithromycine par rapport au poids total de la composition.

6. Préparation odontostomatologique selon la revendication 1, **caractérisée en ce qu'**elle comprend au moins un agent radio-opaque choisi dans le groupe constitué par les polymères acryliques, l'apatite, les sels inorganiques et les dérivés métalliques.

7. Procédé de fabrication de la préparation odontostomatologique selon l'une quelconque des revendications précédentes, comprenant l'étape d'impression 3D de ladite préparation.

8. Préparation odontostomatologique selon l'une quelconque des revendications précédentes, destinée à être utilisée dans la désinfection, la cicatrisation et la régénération des tissus buccaux.
